(19) Europäisches Patentamt
European Patent Office
Office européen des brevets

(11) EP 4 640 212 A1

(12) EUROPEAN PATENT APPLICATION
published in accordance with Art. 153(4) EPC

(43) Date of publication:
29.10.2025 Bulletin 2025/44

(21) Application number: 23904910.9

(22) Date of filing: 22.12.2023

(51) International Patent Classification (IPC):
A61K 9/127 (2025.01)          A61K 31/07 (2006.01)
A61K 47/26 (2006.01)          C12N 15/00 (2006.01)
A61K 47/30 (2006.01)          A61P 3/04 (2006.01)
A61P 35/00 (2006.01)

(52) Cooperative Patent Classification (CPC):
A61K 9/127; A61K 31/07; A61K 47/26;
A61K 47/30; A61P 3/04; A61P 35/00; C12N 15/00

(86) International application number:
PCT/BR2023/050490

(87) International publication number:
WO 2024/130374 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 23.12.2022  BR 102022026572
21.12.2023  BR 102023027077

(71) Applicant: Marjan Industria E Comercio Ltda
Santo Amaro 04755070 São Paulo (BR)

(72) Inventors:
• SIMONE MOLINA, Erika
  05616090 São Paulo (BR)
• MISSAILIDIS, Sotiris
  24350047 Rio de Janeiro (BR)
• JUE LIU, Davi Jing
  04026001 São Paulo (BR)

(74) Representative: Patentwerk B.V.
P.O. Box 1514
5200 BN 's-Hertogenbosch (NL)

Remarks:
The complete document including Reference
Table(s) and the Sequence Listing(s) can be
downloaded from the EPO website

(54) FUNCTIONALIZED LIPOSOMAL COMPLEX, PHARMACEUTICAL COMPOSITION, COSMETIC COMPOSITION AND RELATED USES

(57) The present invention relates to targeted delivery systems of active compounds to adipose tissue, more specifically to white adipocytes cells, or simply white adipocytes. The invention also comprises the funcionalization of liposomes with targeting agents for delivery of active compounds to white adipocytes. More specifically, the present invention addresses the use of liposomes decorated with oligonucleotides, particularly aptamers, for the targeted delivery of active compounds to white adipocytes, with the aim of transforming white adipocytes into brown adipocytes.

The objective of the present invention is to provide a solution to transform the white adipocytes cells into brown adipocytes cells that demonstrates more effective results with reduced or no side effects delivered through therapeutic or cosmetic compositions.

FIG. 2

EP 4 640 212 A1

**Description**

FIELD OF INVENTION

The present invention is within the field of targeted delivery systems of active compounds to adipose tissue, more specifically to white adipocyte cells, or simply white adipocytes. The invention also falls within the field of functionalization of liposomes with targeting agents for delivery of active compounds to white adipocytes. More specifically, the present invention relates to liposomes decorated with nucleic acids, particularly aptamers, for targeted delivery of active compounds to white adipocytes, aiming at the transformation of white adipocytes into brown adipocytes.

Problems and diseases associated with the accumulation of white fat

There are several documented medical conditions and diseases that are directly or indirectly related to the accumulation of fat in adipose tissue, particularly in white adipose tissue (WAT). Some of the diseases and/or medical conditions most frequently associated with such accumulation are obesity, type 2 diabetes (T2DM), heart disease, metabolic syndrome, hyperglycemia, hypertension, steatosis, and some types of cancer, such as endometrial, breast, esophageal, and pancreatic cancer. The most prevalent is obesity, a condition with a multifactorial cause resulting from the interaction of genetic, metabolic, social, behavioral, and cultural factors, causing a strong impact on both health and psychological wellbeing and, consequently, on the individual's quality of life.

Human obesity has become a global pandemic due to sedentary lifestyles and excessive consumption of high-calorie foods rich in saturated fats and sugars. It currently affects one-third of the world's population, including adolescents and children. The World Health Organization estimated that in 2008, 1.5 billion adults aged 20 and over were overweight and more than 500 million men and women were obese.

By 2025, an estimated 2.3 billion adults worldwide will be overweight, with 700 million individuals having a body mass index (BMI) above 30, indicating obesity. In Brazil, this chronic disease has increased by 72% in the last thirteen years, rising from 11.8% in 2006 to 20.3% in 2019. According to the Surveillance Survey of Risk and Protective Factors for Chronic Diseases by Telephone Survey (Vigitel), the frequency of obesity is similar in men and women.

Obesity is a source of reduced performance, restricted work days, absenteeism, lower productivity at work (presenteeism), reduced quality of life, permanent disability, significant morbidity and mortality, and reduced life expectancy. In Brazil, recent data show that the amount spent by the Brazilian Health System (SUS) in 2019 on the care of overweight and obesity was R$1.5 billion. This number represents 22% of the annual direct expenditure on chronic noncommunicable diseases (NCDs) in Brazil. A study presented at the 11th Brazilian Congress of Epidemiology, held in 2021, showed that if the current rate of increase in excess weight continues until 2030, there will be almost 2 million new cases of NCDs and more than 400 thousand deaths due to this problem. Furthermore, a study carried out by the José Alencar Gomes da Silva National Cancer Institute (Inca) reveals that spending on obesity-related cancer cases among adults amounted to R$1.4 billion of the total R$3.5 billion invested in 2018 by the federal government in treating the disease in the Brazilian Health System (SUS) network.

In the United States and Canada, the total annual economic cost of overweight and obesity related to medical costs, excess mortality, and disability was estimated at approximately $300 billion in 2009. International studies of the economic costs of obesity have shown that they account for between 2% and 10% of total health care costs. In addition, voluntary expenditures for weight loss, mostly through unproven means, exceed $75 billion annually.

Currently available symptomatic treatments for obesity fail to achieve long-term therapeutic goals, largely due to the limited efficacy of these medications and poor patient adherence to lifestyle changes and therapies. Few medications are available for the treatment of obesity, and these therapies are primarily aimed at decreasing energy intake by suppressing appetite through central nervous system stimulation or by reducing digestion and absorption of nutrients in the gastrointestinal tract. However, these medications are often accompanied by unpleasant and potentially harmful side effects.

Furthermore, several obesity drugs have been withdrawn from the market due to safety concerns. Small molecules currently in development face considerable challenges in achieving regulatory approval mainly due to their unknown safety profile. To date, only restrictive and malabsorptive bariatric surgery can lead to significant long-term weight loss with some favorable cardiovascular benefits. Consequently, there is an urgent need to develop an effective and safe medical treatment for human obesity and related disorders that significantly reduces healthcare costs, improves quality of life, and saves human lives.

In addition to the issue of the lack of treatment for obesity itself, the public health concern in modern society related to this disease also arises from its association with a broad spectrum of metabolic diseases, including type 2 diabetes (T2DM), heart disease and several types of cancer.

Obesity is characterized morphologically by excessive storage of lipids in white adipose tissue (WAT) and is characterized as a complex disorder of energy imbalance in which energy intake exceeds expenditure. This imbalance leads to the storage of excess energy as fat (triglycerides) in adipocytes, which typically exhibit hypertrophy (increase in cell size) and hyperplasia (increase in cell number or adipogenesis). Excess white fat, a hallmark of obesity, has been shown to increase the risk of type 2 diabetes, high blood pressure, and other diseases.

A less common type of fat, called brown fat or brown adipose tissue (BAT), uses blood sugar and fat molecules to create heat and help maintain body temperature. Studies suggest that a higher presence of BAT may have beneficial effects on health by promoting increased energy expenditure. In this sense, approaches to stimulating thermogenesis to increase the conversion of WAT to BAT have been one of the most recently considered strategies as an alternative to control diabetes, other metabolic diseases related to the accumulation of white fat and reduction of measurements.

Thermogenesis

Thermoregulation is a term that, in biology, refers to the set of systems that regulate the body temperature of some living beings (especially mammals and birds). This regulation is carried out thanks to the coordination between the production (thermogenesis) and release (thermodispersion) of internal organic heat.

The term "thermogenesis" comes from the Greek word thermos for heat. All metabolic processes in animals (and plants) produce heat as a reflection of thermodynamic inefficiency and are therefore thermogenic. In general, however, thermogenesis is used to describe a facultative or adaptive process of heat generation, that is, a process in which heat is the primary product of metabolism. It is also used to describe heat produced in direct response to a meal.

The concept of facultative thermogenesis is well established in relation to the generation of heat to maintain body temperature (thermoregulatory thermogenesis). It is also widely used in nutritional science to describe adaptive heat production in response to overfeeding (diet-induced thermogenesis), particularly as a component of whole-body energy balance regulation.

Brown adipose tissue (BAT) is the primary site of thermogenesis through uncoupling of mitochondrial respiration and ATP synthesis via uncoupling protein 1 (UCP1). When producing heat, BAT consumes not only free fatty acids but also large amounts of glucose, providing benefits for metabolic health. Classical BAT is abundant in neonates but declines with age, leading to limited amounts in adult humans. This compromises the therapeutic value of BAT in the clinical intervention of obesity.

The recent discovery of inducible BAT (iBAT), also called beige adipose tissue, in WAT depots rekindles the promise of treating obesity through stimulation of energy expenditure. Beige adipocytes also possess robust thermogenic capacity.

Current strategies to improve beige adipocyte biogenesis and function involve genetic and systemic pharmacological manipulations, which are associated with significant translational challenges due to the risks of unintended adverse consequences in other cells/tissues and lack of control over the location and temporal extent of beige adipocyte biogenesis. Thus, there is a need to provide an effective delivery system for active compounds capable of inducing beige adipocyte biogenesis in a spatiotemporally controlled and safe manner, avoiding the occurrence of adverse events as much as possible.

In addition to therapeutic applications, the cosmetics and beauty market has been demanding products capable of providing body shaping. In particular, there is great interest in solutions for reducing measurements.

An interesting route for developing solutions in this regard is to bring and adapt products from the pharmaceutical field to the cosmetics field. Obviously, compositions for cosmetic purposes present several other particularities, however, many of the challenges faced during their development are the same as those that arise for pharmaceutical compositions.

It is therefore understood that the development of new solutions aimed at transforming white adipocytes into brown ones finds wide application, both for the pharmaceutical and cosmetic industries. Active compounds that induce the transformation of white adipocytes into brown ones and related to thermogenesis can therefore be used, for example, for the purpose of reducing measurements. Characterization of the technical problem

For a pharmaceutical or cosmetic composition to be effective in producing the desired result, it must meet several requirements. First, the composition must be able to be absorbed by the body to make its active compound available, whether administered orally, topically or intravenously. Next, the composition must reach the target tissue or cell in which the desired biological response must be triggered. Once the target is reached, the active compound in the composition must be effective in triggering the desired biological response. These three steps concern three important properties of a pharmaceutical composition, whether therapeutic or cosmetic: absorption, targeting and potency.

The joint optimization of the three properties mentioned above (absorption, targeting and potency) is a very challenging task, and one that drives the world of R&D in the pharmaceutical and cosmetic industries. It is no coincidence that the funnel for developing new products has drastic reductions in the number of candidates at each new development phase. This is because the initial stage of identifying active compounds with adequate potency is a relatively standardized task that produces a large volume of candidates, while the subsequent stages of optimizing the absorption capacity and targeting of these candidates require lengthy experiments and enormous creative effort.

Along the path that a medication takes through the body, from the time it is ingested or applied until it reaches its target, and even as a result of its effects on the body, it is possible that undesirable effects may occur during or after its use, in which there is a reasonable possibility of a causal relationship between the treatment and the effect called an adverse event. In this sense, the topical route of administration presents itself as an interesting alternative, with the main advantage of avoiding the first-pass effect and improving the subject's adherence to the treatment or use of the product, mainly for cosmetic/aesthetic purposes.

In this context, a promising candidate for the transport and absorption of active compounds has been liposomes of amphiphilic molecules, in particular phospholipids and their derivatives. Liposomes are small, spherical vesicles, preferably composed of phospholipids and generally consist of one or more bilayers surrounding an aqueous interior. Liposomes can encapsulate both hydrophilic drugs (in the aqueous interior) and lipophilic drugs (in the lipid bilayer) and are therefore highly suitable for the development of new pharmaceutical and cosmetic compositions.

Despite the advantages offered, there are important challenges associated with the use of liposomes to encapsulate active compounds. For example, liposomes have restricted targeting capabilities, limited retention and stability in circulation, potential toxicity after chronic administration, and the inability to extravasate. Therefore, important challenges need to be overcome so that a liposome-based pharmaceutical or cosmetic composition can not only encapsulate a desired active compound, but also reach the target tissue efficiently and with sufficient specificity in terms of safety and effectiveness.

In view of the challenges enumerated above, the main objective of the present invention is to disclose embodiments of liposomes decorated with oligonucleotides, particularly aptamers, for targeted delivery of active compounds capable of transforming WAT (white adipose tissue) into BAT (brown adipose tissue).

OBJECTIVES OF THE INVENTION

A first objective of the present invention is to provide a liposomal complex with optimized capabilities for delivery of active compounds targeted at the transformation of white adipocytes into brown adipocytes.

Another objective of the present invention is to provide an efficient therapeutic solution in the treatment of obesity, type 2 diabetes (T2DM), heart disease, metabolic syndrome, diseases related to hyperglycemia and various types of cancer.

It is also an objective of the present invention to provide a cosmetic solution aimed at reducing measurements.

The present invention also aims to provide a pharmaceutical and/or cosmetic composition comprising liposomal complexes for transforming white adipocytes into brown adipocytes. It is the aim of the present invention that such compositions present greater efficiency and that they result in reduced or absent side effects resulting from their application.

BRIEF DESCRIPTION OF THE INVENTION

The present invention relates to a liposomal complex comprising: (i) a liposome, said liposome comprising a bilayer structure formed by amphiphilic molecules, said amphiphilic molecules comprising at least one phospholipid, at least one cholesterol and at least one cationic or ionizable lipid, these preferably being soy phosphatidylcholine or dioleoylphosphatidylethanolamine (DOPE), cholesterol and dioloyltrimethylammonium propane (DOTAP) or analogues of the lipid SM102, respectively, with or without the addition of 2% DMG-PEG 2000; (ii) at least one active compound; (iii) at least one targeting agent coupled to the external portion of the liposome, wherein said at least one targeting agent is an aptamer.

According to one embodiment of the present invention, the aptamer has a sequence with at least 50% identity to SEQ ID NO: 1, SEQ ID NO: 2 and/or SEQ ID NO: 3.

Said aptamers may further comprise a terminal modification consisting of the addition of an amine group NH2, preferably, or a thiol group SH, alternatively.

According to a preferred embodiment of the present invention, the liposome comprises the following amphiphilic molecules: phosphatidylcholine or dioleoylphosphatidylethanolamine (DOPE), cholesterol and cationic or ionizable lipid in a ratio of 45:30:25 (w/w). Preferably, such amphiphilic molecules are soybean phosphatidylcholine or dioleoylphosphatidylethanolamine (DOPE), cholesterol and dioleoyltrimethylammonium propane or analogues of the SM102 lipid. Furthermore, such amphiphilic molecules comprised in the liposome bilayer may present a modification consisting of the addition of an NH2 (amine) group or, more preferably, COOH (carboxyl), or in the presence of 2% DMG-PEG 2000 in its phospholipid portion.

According to a preferred embodiment of the present invention, the liposome comprises the following amphiphilic molecules: phosphatidylcholine or dioleoylphosphatidylethanolamine (DOPE), cholesterol and cationic or ionizable lipid in a ratio of 15-35:30-50:25-45 (w/w). Preferably, such amphiphilic molecules are soybean phosphatidylcholine or dioleoylphosphatidylethanolamine (DOPE), cholesterol and dioleoyltrimethylammonium propane or SM102 and its analogues. Furthermore, such amphiphilic molecules comprised in the liposome bilayer may present a modification consisting of the addition of an NH2 (amine) or COOH (carboxyl) group, or in the addition of 2% DMG-PEG 2000 in its phospholipid portion.

According to a preferred embodiment of the present invention, the at least one active compound is selected from a group of retinoids, more preferably the active compound is an all-trans retinoic acid (ATRA), 9-cis retinoic acid (9-cis RA) or 13-cis retinoic acid (13-cis RA). According to an embodiment of the present invention, the active compound, preferably ATRA, 9-cis RA or 13-cis RA, is present in the liposome in a concentration between 0.001% and 0.4% by mass.

According to a preferred embodiment of the present invention, the active compound, preferably ATRA, is located in the lumen of the liposome bilayer.

According to another embodiment of the present invention, it relates to a pharmaceutical composition comprising the liposomal complex as defined in the previous paragraphs, the composition further comprising at least one pharmaceutically acceptable excipient. According to another embodiment of the present invention, it relates to a targeting compound based on aptamers that present: sequence with at least 50% identity to the sequence SEQ ID NO: 1, that present a sequence with at least 50% identity to the sequence SEQ ID NO: 2, that present a sequence with at least 50% identity to the sequence SEQ ID NO: 3, that present a sequence with at least 50% identity to the sequence SEQ ID NO: 4, which would be coupled to the liposome via amino (NH2) or thiol (SH) modifications at its 5' end.

According to another embodiment, the present invention relates to a cosmetic composition comprising a liposomal complex disclosed herein and at least one cosmetically acceptable excipient. Where the cosmetically acceptable excipient can be chosen from among those known to a person skilled in the art for the purpose of specific application and properties of the cosmetic composition.

According to yet another embodiment of the present invention, this concerns the use of the liposomal complex disclosed herein in the preparation of a medicine for treating disorders and diseases related to the accumulation of white adipose tissue, including obesity, type 2 diabetes (T2DM), heart disease, metabolic syndrome, hyperglycemia, hypertension, steatosis and some types of cancer, such as endometrial, breast, esophageal and pancreatic cancer.

According to another configuration, the present invention deals with the use of the liposomal complex disclosed herein for the preparation of a cosmetic composition for reducing measurements.

BRIEF DESCRIPTION OF THE FIGURES

FIG. 1 illustrates a preferred embodiment of the present invention comprising a liposome containing a retinoid and functionalized with aptamers on its external part.

FIG. 2 illustrates an alternative configuration of the liposome of the present invention, presenting multiple phospholipid bilayers arranged in a concentric manner.

FIG. 3 and 4 illustrate a qualitative analysis of aptamer binding in white adipocytes.

DETAILED DESCRIPTION OF THE INVENTION

The present invention relates to a liposomal complex comprising a liposome containing at least one encapsulated active compound and at least one targeting agent in its external portion.

According to a preferred embodiment of the present invention, the liposomal complex is suitable for the targeted delivery of active compounds to white adipocytes for the conversion of these cells into brown adipocytes. In particular, said conversion of white adipocytes into brown adipocytes aims to reduce the accumulation of fat in adipose tissue, thus configuring a suitable treatment method for diseases and/or medical conditions associated with the accumulation of fat in adipose tissue. Furthermore, said reduction of accumulated fat through the conversion of white adipocytes into brown adipocytes aims to provide solutions for cosmetic and aesthetic purposes.

Definitions

Within the scope of the present invention, the term "thermoregulation" refers to a homeostasis mechanism, since, in the presence of external thermal oscillations, it enables the maintenance of body temperature within defined limits.

Within the scope of the present invention, the term "thermogenesis" refers to the dissipation of energy through the production of heat that occurs in specialized tissues, including brown adipose tissue, beige adipose tissue and skeletal muscle.

Within the scope of the present invention, the term "cosmetic purposes" refers to actions that have as their main objective the improvement of the appearance of an object of interest, which object of interest may be, for example, the human body and its parts. The result obtained from an action with cosmetic purposes is not necessarily restricted to the universe of cosmetics, and other expected or unexpected gains may occur in the health or in other aspects of the object.

Within the context of the present invention, the words "cosmetic" and its derivatives and "aesthetic" and its derivatives are used as synonyms.

Within the scope of the present invention, the term "treatment method" refers to a set of means used to alleviate or cure a disease or to transform something. In the context of medicine, it may be synonymous with therapy. A treatment method may also include biological, surgical, pharmacological, physical, hygienic, psychological means, among others, used to cure, alleviate or shorten a disease or other condition that is not classified as a disease in its own right, but that is intended to be changed/transformed by the object.

Within the scope of the present invention, the term "diseases associated and medical conditions associated with fat accumulation" refers to any disease or other health condition that has its occurrence associated with fat accumulation, most commonly occurring a potential or proven association between such fat accumulation and the onset and/or progression of the condition but not limited to this.

In the scope of the present invention, the term "liposome" refers to vesicles constituted of one or more bilayers of amphiphilic molecules, such as phospholipids, oriented concentrically around an aqueous compartment and that serve as carriers of drugs, biomolecules or diagnostic agents. The stability of liposomes can be affected by chemical, physical and biological factors. The concentric layers that make up liposomes are commonly constituted of phospholipids that organize spontaneously in an aqueous medium or by ultrasound in an aqueous medium in which the component of the solution used (ions, molecules) can fill the cavity inside the liposome. Such structures have attracted the attention of researchers as a promising delivery system due to their biocompatibility, biodegradability, low toxicity, easy preparation, prolonged circulation time, and the ability to extend the shelf life of products. Due to these characteristics, liposomes are considered an excellent form of controlled release of drugs or biologically active substances due to their structural flexibility in the size, composition and fluidity of the lipid bilayer, as well as their ability to incorporate a variety of both hydrophilic and hydrophobic compounds.

Within the scope of the present invention, the term "bilayer" refers to a biological or non-biological construct that is composed of two layers. Said layers may be composed of any component or set of components, depending on the object to which this bilayer is a part.

In the context of the present invention, the term "amphiphilic" refers to the property of molecules that exhibit hydrophilic (soluble in aqueous media) and hydrophobic (insoluble in water, but soluble in lipids and organic solvents) characteristics. For a molecule to exhibit both properties (polar and nonpolar), it must have chains of bonds. These chains are in the form of polymer chains composed of a polar group at one end and a nonpolar group at the other end, with a space-filling polymer chain. These long chains contain molecules with symmetrical charge distribution. The average charge distribution along the chain is symmetrical, making this region nonpolar. The polar head carries a small electrical charge, which makes it soluble in polar solvents, such as water. The uncharged tail is much less soluble in water. They react with each other to form more complex species, which in turn form closed membranous vesicles. The formation of these special structures is responsible for the use of amphiphilic compounds in a large number of applications, such as soaps and detergents.

Within the scope of the present invention, the term "polar molecule" refers to molecules in which the dipole moment is non-zero, i.e., the vectors do not cancel each other out and there is a difference in electronegativity in the molecule, resulting in a charge shift. Even if its structure contains a small nonpolar part, if the molecule has a polar region, it will be considered polar. Polar molecules, also called hydrophilic molecules, are normally soluble in water.

Within the scope of the present invention, the term "nonpolar molecule" refers to molecules in which the sum of the vectors that define the dipole moment cancels each other out, i.e., there is no difference in electronegativity between the atoms. Nonpolar molecules, also called hydrophobic, are practically insoluble in water, but these compounds tend to dissolve in other organic compounds.

Within the scope of the present invention, the term "phospholipid portion" refers to the portion of an object that is composed of phospholipids.

In the context of the present invention, the term "phospholipid" refers to a class of lipids that are one of the main components of the cell plasma membrane. They can also end up forming a bilayer in the plasma membrane, since they have amphiphilic characteristics. The structure of the phospholipid molecule generally consists of two hydrophobic fatty acid "tails" linked by means of a glycerol to a hydrophilic "head" consisting of a phosphate group. The phosphate group can be modified with simple organic molecules.

Within the scope of the present invention, the term "cationic lipids" refers to lipid molecules that, when in solution, have a positive charge in a polar portion, being capable of sequestering negatively charged molecules. Examples of cationic lipids suitable for carrying out the present invention are: dimethylaminoethanecarbamoyl cholesterol, (polyethyleneimine)carbamoyl cholesterol, diguanidinoethyl-aminoethane-carbamoyl cholesterol, oleylamine, stearylamine, dimethyldioctadecyl ammonium bromide, dioleoyltrimethylammonium propane, dioleoyldimethylaminopropane, palmitoyltrimethylammonium propane, distearoyltrimethylammonium propane, myristoyltrimethylammonium propane, dimyristyloxypropyl-dimethylhydroxyethyl ammonium bromide, dilauroylglyceroethylphosphocholine, dioleoylglyceroethyl-phosphocholine, dimyristoylglyceroethylphosphocholine, dipalmitoylglyceroethylphosphocholine and distearoylglycer-

oethylphosphocholine.

In the scope of the present invention, the term "ionizable lipids" refers to lipid molecules that are protonated at low pH, becoming positively charged, but remain neutral at physiological pH. The fact that neutral lipids present less interactions with the anionic membranes of blood cells promotes biocompatibility of lipid nanoparticles, which benefits the delivery of molecules in vivo. According to the present invention, a suitable ionizable lipid is chosen from a group containing: 1,2-dilinoleyloxy-N,N-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl-4-(dimethylamino)butanoate (DLin-MC3-DMA or MC3), 2,2-dilinoleyl-4-(2-dimethylaminoethyl)-[1,3]-dioxolane (DLin-KC2-DMA), 1,2-dioleyloxy-N,N-dimethylaminopropane (DODMA), 2-({8-[(3β)-cholest-5-en-3-yloxy]octyl}oxy)-N,N-dimethyl-3-[(9Z,12Z)-octadeca-9,12-dien-1-yloxy]propan-1-amine (Octyi-CLinDMA), (2R)-2-({8-[(3β)-cholest-5-en-3-iloxy]octyl}oxy)-N,N-dimethyl-3[(9Z,12Z)-octadeca-9,12-dien-1-yloxy]propan-1-amine (Octyi-CLinDMA(2R)) and (2S)-2-({8-[3β)-cholest-5-en-3-yloxy]octil}oxy)-N,N-dimethyl-3-[(9Z,12Z)-octadeca-9,12-dien-1-yloxy]propan-1-amine (Octil-CLinDMA (2S)). Other ionizable lipids may be proposed by a person skilled in the art without the invention departing from the scope of the present patent application.

In the context of the present invention, the term "aptamers" refers to short single-stranded oligonucleotides that fold into three-dimensional structures, in which a specific interaction with a target molecule occurs. Aptamers are usually created through selection from combinatorial libraries, composed of a set of random sequences, although they also occur naturally as part of riboswitches. Aptamers can be used for both basic research and clinical purposes, as they can act as macromolecular drugs. Aptamers can be constructed directly in test tubes, produced by chemical synthesis and are thermostable, and can be easily lyophilized and industrially scaled up, promoting little or no immunogenicity in therapeutic applications. One of the most prominent characteristics of aptamers is their multiplicity of three-dimensional conformations. Due to this flexibility, aptamers have an excellent capacity to bind to other biological macromolecules, especially proteins, and can act as enzyme inhibitors/activators, molecular markers, targeting agents for the delivery of active compounds, among others.

Within the scope of the present invention, the term "truncation" refers to a premature cut of a nucleotide sequence that may lead to a resulting material with preserved or unpreserved function. In this sense, a truncated sequence presents a cut in relation to its most complete presentation form, but the function of the product or products resulting from this action may or may not be altered when compared to the non-truncated form.

Within the scope of the present invention, the term "transport" refers to the ability of a molecule or set of molecules to carry compounds, for example active compounds. The molecule to be transported may be located in the lumen, in the outer layer or in any other component of the carrier.

Within the scope of the present invention, the term "adipogenesis" refers to the process of cellular differentiation in which preadipocytes become adipocytes. Physiologically, it also refers to the production of adipose tissue and the storage of lipids by adipocytes.

In the context of the present invention, the term "lipogenesis" refers to the synthesis of fatty acids and triglycerides, which will subsequently be stored in the liver and adipose tissue. Lipogenesis is regulated by several factors, including nutritional, hormonal and genetic elements. It generally occurs in high-calorie diets with excess carbohydrates and/or proteins.

Within the scope of the present invention, the term "stabilizers" refers to compounds capable of interfering in the physical and chemical degradation processes, in order to improve the stability of a main compound of interest or of processes related to its delivery and metabolization to a target of interest.

Within the scope of the present invention, the term "pharmaceutically acceptable salts" refers to ionic substances resulting from the modification of a non- ionic parent substance providing the corresponding acidic or basic salts, as well as conjugation with amino acids. Pharmaceutically acceptable salts comprise salts of organic or mineral acids with basic residues such as amines; salts of organic or alkaline bases with acidic residues such as carboxylic acids; conventional non-toxic salts comprising those derived from inorganic acids such as hydrochlorides, hydrobromides, hydroiodides, sulfurates, sulfamates, phosphorates and nitrates, not limited to, or those derived from organic acids such as acetates, 2-acetoxybenzoates, ascorbates, benzenesulfonates, benzoates, citrates, ethanesulfonates, ethane disulfonates, formates, fumarates, gentisinates, glycaronates, gluconates, glutamates, glycolates, hydroxymaleates, isethionates, isonicotinates, lactates, maleates, malates, mesylates, oxalates, pamoates, pantothenates, phenylacetates, propionates, salicylates, sulfanilates, toluenesulfonates, stearates, succinates, tartrates and bitartrates, not limited to - as well as quaternary ammonium salts.

Within the scope of the present invention, the term "pharmaceutically acceptable excipient" refers to compounds that have important functions of ensuring the dose, stability, bioavailability and other characteristic aspects of an active compound so that it reaches the targets of interest in the most appropriate manner. The substances used as excipients must present the characteristics necessary for their function, but because they are used in compounds and compositions administered to humans, they must also meet the safety requirements demanded by regulatory agencies and other safety bodies for medicines and cosmetic products.

Within the scope of the present invention, the term "targeting agents" refers to molecules or macromolecules capable of binding with substantial or high specificity to a target. The target to which the targeting agents bind are generally molecules present on the external portion of cells, such as receptors and other membrane proteins. According to the present invention, preferred targeting agents are those capable of binding with substantial or high specificity to white adipocytes. Examples of such targeting agents include aptamers, antibodies, peptides, among other molecules and macromolecules.

Within the scope of the present invention, the term "active compound" refers to molecules capable of triggering some desired therapeutic or cosmetic effect. According to the present invention, the desired therapeutic or cosmetic effect is the induction of transformation of white adipocytes into brown adipocytes, a process also known as browning. An example of an active compound that meets the purposes of the present invention is all-trans retinoic acid (ATRA), in this report also referred to as ATRA. Other examples of active compounds suitable for carrying out the present invention are 9-cis retinoic acid (9-cis RA) or 13-cis retinoic acid (13-cis RA).

Within the scope of the present invention, the term "adverse event" refers to any response to a drug or cosmetic that is harmful or unintended that occurs at doses normally used in humans for prophylaxis, diagnosis, treatment of diseases and cosmetic use, or for the modification of a physiological function.

Within the scope of the present invention, the term "beige adipocyte" refers to brown adipocytes obtained by inducing the transformation of white adipocytes into brown adipocytes. Both beige and brown adipocytes present pronounced thermogenic activity in relation to white adipocytes, their presence being beneficial for the purposes of the present invention. Throughout this descriptive report, the terms "beige adipocyte", "brown adipocyte", "brown adipose tissue" and "BAT" may be read interchangeably.

Within the scope of the present invention, the term "topical administration" is the administration of active compounds/medications locally, most often into intact skin. The active compound/medication is absorbed through the epidermal layer into the dermis. We also have oral administration, where the active compound/medication is administered through the mouth; and intravenous administration, which allows access of an active compound/medication or other solution to the body through direct injection into a vein. For this, it is necessary to perform a venipuncture and use a long needle, capable of penetrating the subcutaneous and muscular tissues to reach the blood vessel.

Within the scope of the present invention, the term "browning" refers to the process of inducing darkening of white adipocytes to brown adipocytes.

Liposomes

According to a preferred embodiment of the present invention, the liposomal complex comprises a liposome with a bilayer structure, preferably constituted by molecules of an amphiphilic nature.

The bilayer structure of the liposomes of the present invention consists of two layers of amphiphilic molecules, with the apolar portion of the amphiphilic molecule facing the interior of the bilayer and the polar portion facing outside the bilayer. In this arrangement, the apolar portion of the amphiphilic molecules is enclosed within the bilayer, not being exposed to the exterior of the liposome or to the interior of the liposome (also called the liposomal lumen), while the polar portion of the amphiphilic molecules is exposed to the exterior of the liposome and to the interior of the liposome (also called the liposomal lumen). Thus, the bilayer structure of the present invention becomes suitable for the encapsulation of polar active compounds and for the encapsulation of apolar active compounds in the interior portion of the bilayer. This same bilayer structure is favorable for the entrapment of other nonpolar compounds within the bilayer, which may have a function as active compounds or any other function to optimize the therapeutic or cosmetic efficiency of the liposomal complex, such as absorption optimization.

The bilayer structures of the present invention resemble the structures of the cell membranes of eukaryotic organisms and may even accept the incorporation of macromolecules, such as proteins.

Second alternative configuration, the liposome is made up of a monolayer of amphiphilic molecules, which can assume two distinct configurations: (i) with the polar portion facing the lumen of the liposome and the apolar portion facing the exterior of the liposome; or (ii) with the apolar portion facing the lumen of the liposome and the polar portion facing the exterior of the liposome.

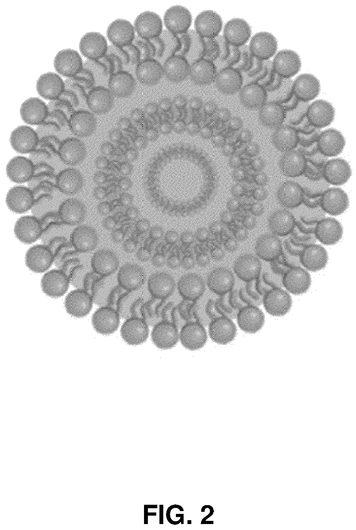

According to another alternative configuration, the liposome may assume a multilayer liposome configuration, comprising one or more layers of amphiphilic molecules. One possible configuration consists of a liposome with two bilayers, one encapsulated within the other. An example of this configuration is illustrated in Figure 2.

According to the preferred configuration of the present invention, the liposomes have an average diameter between 20 nm and 5 μm.

Liposome size affects its biodistribution, since after subcutaneous administration, liposomes smaller than 120 nm readily pass through capillaries, whereas liposomes of approximately 200 nm or larger tend to remain at the injection site. Single-layer liposomes (also called monolayer or unilamellar) vary in size, with the largest (LUV - Large Unilamellar Vesicles) having aspect ratios ranging from 100 to 1000 nm, and the smallest (SUV - Small Unilamellar Vesicles) generally having sizes less than 100 nm.

Amphiphilic molecules

The liposome of the present invention should preferably be constituted by amphiphilic molecules. The use of this type of molecule favors the formation of bilayer structures suitable for the encapsulation of active compounds within the bilayer or in the lumen of the liposome.

According to one embodiment of the present invention, the liposome comprises at least one type of amphiphilic molecule. Preferably, the liposome comprises at least two types of amphiphilic molecules. Even more preferably, the liposome comprises at least three types of amphiphilic molecules.

Liposomes are basically composed of phospholipids, sterols and an antioxidant. Most of these molecules have an amphiphilic character. Within the scope of the present invention, the molecules that constitute the liposome, which may be amphiphilic, may be chosen from the group comprising: phosphatidylcholine, phosphatidylserine, phosphatidylglycerol, phosphatidylethanolamine and sphingomyelin. And among the phosphatidylcholines we have: distearoylphosphatidylcholine, dipalmitoylphosphatidylcholine, dioleylphosphatidylcholine, dimyristoylphosphatidylcholine; among the phosphatidylethanolamines we have: dioleylphosphatidylethanolamine and distearoylphosphatidylethanolamine ; among the phosphatidylglycerols we have: dimyristoylphosphatidylglycerol and dipalmitoylphosphatidylglycerol; among the phosphatidylserines we have: dipalmitoylphosphatidylserine and distearoylphosphatidylserine. Other molecules used for liposome construction are: cholesterol, dioleoyl-trimethylammonium propane, oleylamine, stearylamine, dimethylaminoethane-carbamoyl cholesterol, non-ionic surfactants, ethanol, monosialoganglioside, phosphatidylinositol, polyethylene glycol (PEG), and the lipid derivative of polyethylene glycol, being methoxyPEG with distearylphosphatidylethanolamine mPEG-DSPE.

According to a preferred configuration, at least one of the amphiphilic molecules of the liposome comprises a phospholipid portion, which may consist of one or more phospholipid chains.

According to the preferred embodiment of the present invention, the liposome comprises at least one phospholipid. In general, phospholipids are constituted, in their apolar part, by two hydrophobic chains of fatty acids that may have different lengths and unsaturations. The polar part consists of a phosphate group linked to other polar groups containing charges or not. Phospholipids, when dispersed in aqueous solution, spontaneously organize themselves into bilayers, presenting hydrophobic and hydrophilic regions, in which hydrophilic molecules (in the aqueous compartment), hydrophobic molecules (inside the lipid bilayer) or amphiphilic molecules (partially intercalated in the different regions) can be inserted. Liposomes, because they are biocompatible and biodegradable, are excellent systems for drug delivery in the human body. Liposomes have shown good functionality both in the transport of molecules and in interaction with cell membranes due to their similarity to biological lipid membranes.

According to a preferred embodiment of the present invention, the liposome is of the bilayer type and comprises a phospholipid, in addition to cholesterol and a cationic and/or ionizable lipid, more preferably these are, respectively, soybean phosphatidylcholine or dioleoylphosphatidylethanolamine (DOPE), cholesterol and dioleoyltrimethylammonium propane or SM102 and its analogues. More preferably, the bilayer liposome of the present invention comprises only soybean phosphatidylcholine or dioleoylphosphatidylethanolamine (DOPE), cholesterol and a cationic lipid, preferably dioleoyl-trimethylammonium propane.

The liposome of the above configuration has the components soybean phosphatidylcholine, cholesterol and cationic lipid in the ratio of about 45:30:25 (w/w), respectively. Furthermore, the liposome of the above configuration has the components soybean phosphatidylcholine or dioleoylphosphatidylethanolamine (DOPE), cholesterol and dioleoyl-trimethylammonium propane in the ratio of about 15-35:30-50:25-45 (w/w).

According to an alternative embodiment of the present invention, the liposome is of the bilayer type and comprises a phospholipid, in addition to cholesterol and an ionizable lipid. More preferably, the phospholipid is soybean phosphatidylcholine or dioleoylphosphatidylethanolamine (DOPE) and the ionizable lipid is SM102 and its analogues. More preferably, the bilayer liposome of the present invention comprises only soybean or egg phosphatidylcholine, cholesterol and an ionizable lipid, preferably SM102 and its analogues.

In addition to amphiphilic molecules, the liposome may comprise polar or nonpolar molecules. The incorporation of such molecules may be done with the aim of modifying the properties of the liposome, such as lipids, antioxidants and surfactants.

Modifications can be via structural characteristics of the lipids such as the presence of unsaturation in the nonpolar regions, degree of symmetry and presence of charges in the polar regions that confer properties to modulate the molecular packing parameter, the permeability of the liposomes and the release kinetics of the active compounds.

Some variations of liposomes are:

Long-circulating liposomes in vivo are obtained by different methods, including surface ligands with natural hydrophilic components, or synthetic hydrophilic polymers, specifically polyethylene glycols (PEG).

Modified liposomes are obtained by adding ligands to their surface to increase the specificity of interaction with target cells and the amount of the active substance released into the cells. A large number of ligands have been studied, including peptides, antibodies, proteins, polysaccharides, glycolipids, glycoproteins and lecithins.

It is also conceivable that the liposome incorporates other macromolecules into its bilayer structure, such as membrane proteins, antibodies, aptamers or "small molecules" such as folates.

The molecules that make up the liposome can also be subjected to modifications aimed at altering their molecular properties, such as rotational steric freedom, polarity, addition of reaction groups, among others.

According to a preferred embodiment of the present invention, the amphiphilic molecules comprising the phospholipid portion that composes the liposome are modified so as to preferably receive a carboxyl group COOH or, alternatively, an amine group NH2 or thiol group SH in the polar terminal portion of its phospholipid chain. By adding such groups, it is possible to couple targeting agents to the external portion of the liposome. For example, targeting agents preferably comprising amine groups NH2 or, alternatively, carboxyl groups COOH, can be linked to the liposome by forming a peptide bond with, respectively, the carboxyl group COOH or amine group NH2 of the molecules that compose the liposome. Alternatively, targeting agents comprising thiol SH groups can be attached to the liposome through formation of disulfide bonds with the thiol SH group of the molecules that make up the liposome, such as DMG-PEG 2000.

To couple the aptamer to the nanoparticle, it must be activated by TCEP. To do this, mix 100 $\mu$l of 83.7 mM TCEP solution (24 mg of TCEP in 1 ml of water) with 100 $\mu$l of water and 20 $\mu$l of 2 mM aptamer. This mixture must be placed at 57°C under stirring at 200 rpm for 1 hour. After this time, purify the aptamer in the most convenient way and quantify it by Ellman's method. After activation of the aptamer, functionalization is performed on the lipid nanoparticle containing PEG-maleimide. For this step, the ratio of 5 mg of nanoparticle to 0.6 mg of aptamer in 20 mM phosphate buffer pH 7.0 is used. After mixing, leave to stir at room temperature for 2 hours, then separate the unreacted aptamer by diafiltration with an amicon tube with the necessary cut to avoid the nanoparticle passing through. Finally, recover the functionalized nanoparticle and quantify the aptamer that came out of the amicon using Ellman's method.

The methods for modifying phospholipid chains are widely known in the state of the art, and their description in this report is irrelevant.

Alternatively, the molecules that make up the liposome can be modified to enable the coupling of targeting agents to the external portion of the liposome using other methodologies. Examples of modifications of such molecules to enable coupling are the biotin-streptavidin system. Other systems can be applied without escaping the scope of protection of the present invention.

Active compounds

A liposomal structure, to induce the transformation of WAT into BAT, needs to carry active compounds endowed with this capacity. According to a preferred embodiment of the present invention, said active compound is preferably a retinoid, even more preferably all-trans retinoic acid (ATRA), 9-cis retinoic acid (9-cis RA) and/or 13-cis retinoic acid (13-cis RA).

Retinoids are a class of compounds consisting of four isoprenoid units joined head-to-tail. Retinoids include all-trans retinoic acid (tretinoin), cis-isomeric retinoic acids such as 13-cis-retinoic acid (isotretinoin) and 9-cis-retinoic acid (alitretinoin), vitamin A (retinol), 4-[1-(5,6,7,8-tetrahydro-3,5,5,8,8-pentamethyl-2-naphthalenyl)ethenyl]benzoic acid (bexarotene, Targretin®), retinal, retiferol, (E,E,E)-7-(2-n-propoxy-5,6,7,8-tetrahydronaphthalen-3-yl)-6-fluoro-3-methyl-locta-2,4,6-trienoic acid, adapalene (6-[3-(1-adamantyl)-4-methoxyphenyl]-2-naphthoic acid), acyclic retinoid [(2E,4E,6E,10E)-3,7,11,15-tetramethylhexadeca-2,4,6,10,14-pentaenoic acid], ethyl (E,E,E)-7-(2-n-propoxy-5,5,8,8-tetramethyl-5,6,7,8-tetrahydronaphthalen-3-yl)-6-fluoro-3-methylocta-2,4,6-trienoate, and their natural and synthetic derivatives. Retinoids are essential for many life processes, including vision, reproduction, metabolism, differentiation, bone development, and pattern formation during embryogenesis.

Retinoids can be administered orally or applied to the skin, and their most traditional application is related to the treatment of dermatological conditions, such as acne and psoriasis. Some studies also prove their benefits in skin aging.

In addition to the cutaneous effects, among the effects caused by retinoids, it is known that, when internalized into the cytoplasm of adipocyte cells, their active biological form, retinoic acid (RA), is internalized into the nucleus through the RARs (retinoic acid receptors) and RXRs (retinoid x receptors). Currently, it is known that these receptors are phosphorylated at the serine residue (Ser 369 for RAR) after activation of PKA (protein kinase A) and, in the nucleus, RA triggers gene transcription by binding to heterodimers formed by RA receptors (RAR$\alpha$, RAR$\beta$ and RAR$\gamma$) and retinoid X receptors (RXR$\alpha$, RXR$\beta$ and RXR$\gamma$).

These two major groups of receptors belong to the large family of nuclear receptors for steroid hormones. Upregulation of genes at the mRNA levels of UCP1, UCP2, respiratory chain components and transcription factors (PGC-1$\alpha$, PPAR$\alpha$) and enzymes (CPT1) involved in fatty acid oxidation in different deposits of white adipose tissue (WAT) is consistent with an increased capacity for oxidative metabolism and thermogenesis. More specifically, more favorable metabolic effects on brown adipose tissue (BAT) transformation and thermogenesis by all-trans retinoic acid (ATRA), 9-cis retinoic acid (9-cis RA) and/or 13-cis retinoic acid (13-cis RA) have been demonstrated in the literature, presenting advantages in binding and activation of RARs receptors and availability compared to 9-cis-retinoic acid and bexarotene.

All-trans retinoic acid (ATRA), 9-cis retinoic acid (9-cis RA) and/or 13-cis retinoic acid (13-cis RA) are known as some of the most active forms of vitamin A, participating in the regulation of proliferation, differentiation and apoptosis of various cell types. These metabolites have a direct influence on the biology of adipocytes, acting in the weight loss process by reducing the processes of adipogenesis and lipogenesis.

In alternative embodiments, the present invention may include active compounds other than retinoic acid, particularly ATRA, 9-cis RA and/or 13-cis RA. There are several agents with the potential to induce thermogenesis and transform WAT into BAT (Table 1).

Table 1. Active compounds related to the induction of WAT transformation into BAT.

| COMPOUNDS | MECHANISM OF ACTION |
|---|---|
| Retinoic acid | Retinoic acid induces WAT browning through activation of vascular endothelial growth factor (VEGF) signaling *in vitro* |
| Cold exposure | Cold exposure stimulates BAT insulin signaling in rats |
| CL 316243 | Chronic treatment with CL 316243 leads to the occurrence of brown adipocytes in traditional WAT deposits |
| BRL 26830A | BRL 26830A treatment increases UCP1 expression in periovarian WAT deposits in rodents |
| Acetate | Acetate treatment shows browning ability of WAT in mice |
| Capsaicin | Capsaicin initiates WAT browning in mice by several different mechanisms |
| Resveratrol | Resveratrol induces UCP1 and Pgc1$\alpha$ gene expression in mice |
| Berberine | Berberine induces brown adipocyte development in the inguinal WAT deposit in mice |
| Fish oil | Fish oil intake leads to upregulation of UCP 1 and $\beta$3 adrenergic receptor in mouse inguinal WAT |
| Decaffeinated Green Tea Extract | Decaffeinated green tea extract induces the expression of a variety of biomarkers related to adipose tissue browning |
| Cinnamon | Cinnamon affects UCP1 expression in adipocytes isolated from mouse subcutaneous WAT |
| Ginsenodisis Rb1 curcumin quercetin | Ginsenoside Rb1 , curcumin and quercetin promote mRNA expression of BAT markers in 3T3-L1 cells |
| Farnesoid x receptor | Farnesoid X receptor activation increases thermogenesis and browning of subcutaneous WAT |
| Liver X receptors | Liver X receptors regulate WAT browning, mitochondrial activation, and increased energy expenditure |
| MicroRNAs | MicroRNAs regulate BAT activation and subcutaneous WAT browning upon cold exposure |
| Thiazolidinediones | Chronic treatment with thiazolidinediones (TZDs) induces WAT browning by activating PPAR$\gamma$ and PRDM16 |
| Prostaglandin E2 | Prostaglandin E2 diverts the differentiation of WAT preadipocytes to beige adipocytes |
| Gleevec | Gleevec increases thermogenic and mitochondrial genes of brown/beige fat in WAT and interscapular BAT in mice |
| beta-lapachone | Beta- lapachone stimulates WAT browning and increases UCP1 expression in high-fat diet-fed mice |
| Slit-derived secretory product 2 | Slit-derived secretory product 2 regulates beige adipocyte induction via PRDM16 and activation of PKA signaling |
| Artepillin C | Artepillin C induces brown adipocytes in mice and adipocytes derived from primary inguinal white adipose tissue (WAT) *in vitro* |
| Adrenomedullin 2 | Adrenomedullin 2 induces browning of primary rat adipocytes *in vitro* |

(continued)

| COMPOUNDS | MECHANISM OF ACTION |
|---|---|
| IL-6 | Bone marrow-derived IL-6 during burn injury regulates the browning of WAT |
| IL-4 | IL-4 plays a macrophage-dependent role in the activation of BAT during cold exposure |
| IEX-1 | IEX-1 deficiency leads to WAT browning through alternative macrophage activation. |
| Thyroid hormones | The thyroid hormone metabolite TRIAC induces UCP1 expression in abdominal WAT of rats; thyroid hormones induce WAT browning via both peripheral and central mechanisms |
| Glucagon-like peptide-1 (GLP-1) | GLP-1 stimulates WAT browning and promotes BAT thermogenesis in mice |
| Leptin | Leptin drives WAT browning through its action on hypothalamic neurons |
| Melatonin | Melatonin induces darkening of WAT in inguinal WAT in rats |
| Natriuretic peptides | Natriuretic peptides demonstrate WAT browning ability with various different mechanisms |
| PTEN Cox2 Foxc2 Folliculin Gq | A distinct set of genes is associated with WAT browning, including PTEN, Cox2, Foxc2, folliculin and Gq |
| TGF-β/Smad3 | Modulations in TGF-β/Smad3 signaling activate BAT-like phenotype in rodent WAT |
| FGF21 | FGF21 expression is essential for cold-induced recruitment of beige adipocytes |
| Apelin | Apelin secreted from brown and white adipocytes stimulates adipose browning |
| PPAR agonists | Treatment with PPAR agonists increases UCP1 expression in different WAT deposits |
| BMPs | BMP7 can induce selective BAT genes in preadipocytes and drive browning of mesenchymal progenitor cells |
| Lactate β-hydroxybutyrate | Lactate and β-hydroxybutyrate increase UCP1 gene expression in human and murine WAT |
| Beta- aminoisobutyric acid (BAIBA) | BAIBA increases BAT marker genes in mouse WAT *in vitro* and *in vivo* and BAT-like phenotype in human pluripotent stem cells |

The wide range of possibilities of active compounds available to obtain the result intended by the present invention demonstrates the intellectual effort associated with the selection process for a final composition that is truly functional and effective as revealed by the present invention.

In a preferred embodiment of the present invention, the active compound is used in a concentration between 0.005 uM to 150 uM of ATRA in a specific vehicle.

In an alternative configuration, the active compound transported by the liposomal structure, as per possible configurations for the present invention, may be combined with stabilizers, with one or more active compounds and/or with pharmaceutically acceptable salts.

Targeting agents

Targeting agents are molecules with the ability to selectively bind to a specific tissue or cell. In general, this binding occurs between a targeting agent and a cellular protein located on the external portion of the cell, such as cellular receptors. Binding can occur through the combination of multiple molecular forces, such as charge interactions, dipole interactions, three-dimensional steric docking, Van Der Waals interactions, hydrogen bonds, π electron stacking, hydrophobic interactions, among others. Aptamers prefer to interact with positively charged surfaces of target proteins due to the negatively charged nature of the backbone bonds of the phosphate groups.

According to a preferred embodiment of the present invention, the targeting agent is an oligo(deoxy)ribonucleotide. Examples of suitable oligo(deoxy)ribonucleotides are single-stranded DNA (ssDNA) and single-stranded RNA (ssRNA). According to an even more preferred embodiment, the targeting agents of the present invention are ssDNA aptamers.

Aptamers suitable for use as targeting agents are those capable of selectively binding to adipocyte receptors, especially white adipocytes. Selective binding of the aptamer to the cell can occur through the interaction of the aptamer with receptors or other proteins located on the outer portion of the cell membrane.

The selection of suitable aptamers for selective binding to white adipocytes can be conducted by techniques known in the technical field, such as SELEX (acronym for the English term Systematic Evolution of Ligands by Exponential Enrichment). SELEX experiments can be conducted against a variety of target molecules or elements, such as small compounds, proteins, nanoparticles or living cells.

According to the present invention, suitable white adipocyte membrane proteins and receptors as targets for selective binding of aptamers and other targeting agents are: adipocyte plasma membrane associated protein (APMAP), Aldosterone synthase mRNA (CYP11B2), among others associated with nuclear proteins.

An example of an aptamer suitable for use as a targeting agent within the scope of the present invention is the ssDNA aptamer with the sequence SEQ ID NO: 1. According to an embodiment of the present invention, aptamers which have at least 30% identity with the sequence SEQ ID NO: 1 are also suitable, preferably at least 50%, more preferably 70%, even more preferably at least 90%.

Another example of an aptamer suitable for use as a targeting agent within the scope of the present invention is the ssDNA aptamer with sequence SEQ ID NO: 2. According to an embodiment of the present invention, aptamers that have at least 30% identity with the sequence SEQ ID NO: 2 are also suitable, preferably at least 50%, more preferably 70%, even more preferably at least 90%.

It is conceivable that the aptamers used in the present invention are aptamers described in the state of the art. Examples of aptamers described in the literature and which would be suitable for incorporation in the present invention as targeting agents and, optionally, also as active compounds are: adipo-8 and adipo-1.

According to an alternative embodiment of the present invention, at least two, or even more than two, targeting agents may be coupled to the external portion of the liposome. Preferably, the at least two or more than two targeting agents are all aptamers. Alternatively, at least one of the at least two or more targeting agents is an aptamer, and the others may be other types of molecules, such as proteins, antibodies and any other molecule or macromolecule capable of acting as a targeting agent to achieve the purposes of the present invention.

According to an alternative configuration of the present invention, the aptamers coupled to the external portion of the liposome perform therapeutic and cosmetic functions. In particular, such aptamers are effective in converting white adipocytes into brown ones. In view of such therapeutic and cosmetic effectiveness, an alternative configuration of the present invention is conceivable in which the liposomal complex comprises a bilayer structure decorated with aptamers in its external portion, as disclosed in this report, without comprising any active compound. In this configuration, the targeting agent itself acts as the active compound, being responsible both for the specificity of binding to the specific tissue, cell or receptor, and for producing the desired therapeutic effect. According to such configuration, the aptamers, preferably an aptamer with sequence SEQ ID NO: 1, coupled to the external portion of the liposome are capable of binding with substantial specificity to white adipocytes, triggering their transformation into brown adipocytes and, thus, promoting the desired therapeutic or cosmetic effect.

Permeability agents

According to the present invention, the liposomal complex may further comprise permeability agents, the function of which is to increase the absorption of the complex through the skin.

Within the scope of the present invention, permeability agents comprise aptamers, proteins and chemical compounds capable of conferring greater permeability of the liposomal complex, or part or components thereof, through the skin, so as to allow delivery of the active compound to the target tissue or cell.

According to a preferred embodiment of the present invention, the permeability agents consist of aptamers, preferably an aptamer with a nucleotide sequence with at least 60% identity, more preferably 75% identity, even more preferably at least 90% identity with at least one of the nucleotide sequences defined in SEQ ID NO: 6, SEQ ID NO: 7, SEQ ID NO: 8 or SEQ ID NO: 9.

Aptamer-type permeability agents are preferably coupled to the external portion of the liposomal complex via amino (NH2) or thiol (SH) modifications at their 5' end. According to this configuration, aptamers acting as permeability agents are coupled to the external portion of the liposomal complex together with aptamers acting as targeting agents.

According to an alternative embodiment of the present invention, at least two distinct permeability agents are coupled to the external portion of the liposomal complex, preferably two aptamers selected from the group comprising SEQ ID NO: 6, SEQ ID NO: 7, SEQ ID NO: 8 or SEQ ID NO: 9.

Chemical modifications applied to aptamers

The conformational diversity and targeting specificity of RNA and DNA aptamers provide promising potential for their therapeutic applications as inhibitors of protein-protein interactions and for targeting liposome-based delivery systems, as envisioned by the present invention. To achieve this goal, several aptamer characteristics must be considered. First, aptamers must be highly stable in the body. Since natural RNA and DNA are susceptible to endogenous nucleases, chemical modifications to the sugar and/or phosphodiester backbone must be adequately included to avoid aptamer degradation. Second, the nucleotide length must be sufficiently reduced. Truncating the aptamer will reduce the manufacturing cost of the final pharmaceutical composition, facilitate material quality assurance, and avoid unexpected toxicity. Third, therapeutic aptamers must have good pharmacokinetics, as seen for other drugs.

The following are examples of chemical modifications applied to suitable aptamers according to the present invention. The examples below should not be taken as limiting the present invention, but rather as illustrative examples of the possibilities of chemical modification. A person skilled in the art will know how to choose among the most suitable modifications, whether these are mentioned in this descriptive report or not.

A first form of aptamer modification is the one applied to the sugar of the nucleotides, being the ribose sugar in the case of RNA aptamers and deoxyribose in the case of DNA aptamers. Ribose and deoxyribose modifications at the 2' position (2'-amino, 2'-fluoro and 2'-O-methyl) are widely used to confer nuclease resistance. Such modifications can be incorporated during the chemical synthesis of (ribo)nucleotides. The modified sugars can also be incorporated using mutant T7 polymerase enzymes, such as the T7 RNA polymerase with the Y639F mutation for which the ability to incorporate 2'-fluoro and 2'-deoxypyrimidine during the synthesis of ssRNAs has been reported. Similarly, the addition of two more substitutions in T7 RNA polymerase, H784A and K378R, allows the production of 2'-O-methyl RNA.

Another type of modification refers to those directed at the pyrimidine of (deoxy)ribonucleotides.

Another modification consists of changing the phosphodiester bonds to phosphorothioate and boranephosphate bonds. Polymerization of nucleotides containing such bonds can be carried out with modified Taq DNA polymerase or T7 RNA polymerases.

Alternatively, aptamers can be produced from mirror-image oligonucleotides. The fact that these consist of mirror-image molecules prevents them from being recognized by plasma nucleases. Aptamers made from l-ribose-based nucleotides instead of natural d-nucleotides confer nuclease resistance and are called Spiegelmers. Spiegelmers are selected using mirror-image targets instead of the l-nucleotide library, which is incompatible with enzymatic manipulations. The identified d-aptamers are produced in their respective mirror images from l-nucleotides. The resulting Spiegelmers in turn bind natural targets.

Yet another applicable modification concerns the addition of (ribo)nucleotide sequences to the 3' terminal portion of aptamers, called end-capping. After selecting an aptamer sequence, nuclease resistance can be enhanced by 3' end-capping. Inverted deoxythymidine (idT), which has a 3'-3' linkage, is widely used to prevent 3'-5' exonuclease activity. Locked nucleic acid (LNA) analogues can also be ligated by terminal deoxynucleotidyl transferase, providing nuclease resistance.

The sequence length of aptamers is also a modifiable point. For therapeutic and cosmetic applications, aptamers should be shortened as much as possible. Truncation reduces material cost and unexpected interactions. Furthermore, truncated aptamers are advantageous in aptamer discovery due to their reduced number of modifiable nucleotides for nuclease resistance.

Aptamers can be modified to be stable in the body, to be resistant to nucleases, and to avoid systemic clearance, such as renal filtration. Polyethylene glycol (PEG) attachment is a widely used strategy in the technical field. PEGylation of aptamers decreases their glomerular filtration rate by simply increasing their molecular size. In line with this notion, the SELEX procedure using a PEGylated library has been reported. In this method, aptamers are transcribed with 5'-amino-GMP and then coupled with N-hydroxysuccinimide (NHS)PEG. Therefore, a PEGylated aptamer will be obtained directly through SELEX. A PEGylated library is beneficial to eliminate non-PEGylated candidates, which share a target binding surface and the PEGylation site. Furthermore, PEGylated SELEX allows in vivo selection.

Also noteworthy is the possibility of using aptamers containing artificial base pairs, i.e., additional pairs beyond the traditional AT(U) and CG. Several artificial nucleotides have been developed in the last decade. Artificial nucleotides have the potential to expand aptamer activity. Unnatural bases and unnatural ribose phosphate can alter the target preference of an aptamer rather than providing nuclease resistance. Permissive polymerases are again beneficial for incorporating artificial NTP into an aptamer from the early discovery stage. Aminoallyl uridine is widely used as a modified base. Aminoalil-UTP is acceptable for T7 RNA polymerase. Creating new base pairs is a powerful way to increase aptamer diversity. Aptamers using the base pair 7-(2-thienyl)-imidazo[4,5-b]pyridine(Ds)-2-nitro-4-propynylpyrroyl (Px) have been reported. These incorporated Ds into the aptamer strand and used Px on the counter strand for PCR amplification. Deep Vent DNA polymerase can accept both artificial nucleotides. Sefah et al. reported the AEGIS (Artificially Expanded Genetic Information System)-SELEX method using six letters, i.e., GACTZP. While the Ds-Px bases pair by form-fitting, the ZP pair uses hydrogen bonding. AEGIS includes 12 nucleotides/letters of the base pairs A-T, C-G, S-B, Z-P, V-J and K-X. It was shown that eight previous letters, i.e. ATCGSBZP, could behave as DNA and surprisingly be transcribed into RNA by a mutant T7 RNA polymerase. Therefore, the use of these expanded base pairs for aptamers is quite interesting and can be applied to the present invention.

Therapeutic applications

Brown or beige adipocytes have attracted interest in health sciences due to their ability to counteract various metabolic diseases, including obesity and type 2 diabetes. Thermogenesis without ATP production, orchestrated by UCP1, is an effective way to help reduce energy waste, thus creating a negative energy balance. While some metabolic disorders and obesity are commonly associated with type 2 diabetes, white adipose tissue directly contributes to the onset and persistence of the disease. Resistance to obesity has also been associated with the activation of these tissues in several mouse models.

Furthermore, brown adipose tissue may exert a beneficial effect on whole-body metabolism through other pathways, such as the elimination of lipids and glucose from the circulation. Considering the beneficial effects of the occurrence of BAT, the emergence of beige adipocytes capable of thermogenesis and the darkening of WAT are of interest and potential for the treatment of type 2 diabetes and obesity, among other conditions.

The use of compounds capable of inducing the transformation of white adipocytes into beige adipocytes and, therefore, related to the stimulation of thermogenesis, can also occur in the field of cosmetics, particularly applied to reducing measurements.

Forms of administration

An alternative method of drug administration is topical administration. Topical administration of drugs has several advantages. This method avoids the metabolism in the liver and gastrointestinal tract that oral drugs undergo. The topical route of administration is therefore an interesting alternative in terms of avoiding, for example, adverse events, with the additional advantage of improving the subject's adherence to therapy or to the use of the product for mainly cosmetic purposes.

In this context, a promising candidate for the transport and absorption of active compounds has been phospholipid liposomes and their derivatives. Liposomes are small, spherical vesicles composed of phospholipids and usually consist of one or more lipid bilayers surrounding an aqueous interior. Liposomes can encapsulate both hydrophilic drugs (in the aqueous interior) and lipophilic drugs (in the lipid bilayer) and are therefore highly suitable for the development of new pharmaceutical and cosmetic compositions.

For the liposomal complex of the present invention to be adequately administered, it is conceivable that it be inserted into a pharmaceutical composition, being added together with pharmaceutically acceptable excipients.

Detailed Description of Figures

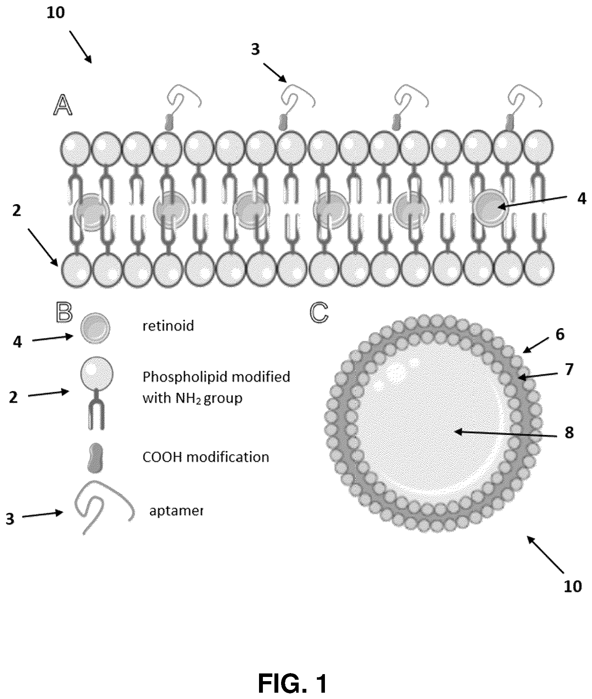

Figure 1 illustrates a preferred embodiment of the present invention, consisting of a liposomal bilayer structure comprising phospholipids. Said bilayer contains an active compound of the retinoic acid type, preferably ATRA, 9-cis RA and/or 13-cis RA, in the lumen of the bilayer 7, i.e., intercalated between the two layers of the phospholipid bilayer. Said liposome is further decorated with targeting agents in its external portion 6, preferably aptamers 3, even more preferably the aptamer with sequence SEQ ID NO: 1.

Figure 1A illustrates the bilayer structure of the liposomal complex according to a preferred embodiment of the present invention. As illustrated, the bilayer structure consists of a single bilayer formed by phospholipids. Said phospholipids having their polar portion facing the external portion 6 preferably modified by the addition of COOH groups.

The liposome 10 of the present invention preferably comprises phospholipids and amphiphilic molecules, these being soybean phosphatidylcholine, cholesterol and cationic lipid in the proportion of around 45:30:25 (w/w), respectively. Preferably soybean phosphatidylcholine, cholesterol and dioleoyl-trimethylammonium propane, preferably cationic, in the proportion of around 15-35:30-50:25-45 (w/w).

Alternatively, the liposome 10 of the present invention comprises an ionizable lipid in substitution for or in combination with the cationic lipid. When in substitution for the cationic lipid, the liposome 10 comprises phospholipids and amphiphilic molecules, these being soy phosphatidylcholine, cholesterol and ionizable lipid in the ratio of about 15-35:30-50:25-45 (w/w), respectively.

Furthermore, the liposome 10 of the present invention has an average diameter between 20 nm and 5 $\mu$m.

Additionally, the liposomal complex comprises targeting agents, more preferably aptamers 3. Such aptamers 3 are also preferably modified by the addition of an amine group NH2 at a terminal portion, so that it binds to the phospholipids of the bilayer of the liposome 10 via reaction between said NH2 group of the aptamers 3 and the COOH group of the phospholipids.

Alternatively, aptamers 3 are modified for coupling the aptamer to the nanoparticle, which must be activated by TCEP. To do this, mix 100 μl of 83.7 mM TCEP solution (24 mg of TCEP in 1 ml of water) with 100 μl of water and 20 μl of 2 mM aptamer. This mixture must be placed at 57°C under stirring at 200 rpm for 1 hour. After this time, purify the aptamer in the most convenient way and quantify it by Ellman's method. After activation of the aptamer, functionalization is performed on the lipid nanoparticle containing PEG-maleimide. For this step, the ratio of 5 mg of nanoparticle to 0.6 mg of aptamer in 20 mM phosphate buffer pH 7.0 is used. After mixing, leave to stir at room temperature for 2 hours, then separate the unreacted aptamer by diafiltration with an amicon tube with the necessary cut to avoid the nanoparticle passing through. Finally, recover the functionalized nanoparticle and quantify the aptamer that came out of the amicon using Ellman's method.

According to a preferred embodiment of the present invention, aptamer 3 is a DNA sequence comprising SEQ ID NO: 1. According to another embodiment of the present invention, the aptamer comprises a sequence with at least 30% identity to SEQ ID NO: 1, preferably at least 50%, more preferably 70%, even more preferably at least 90%.

According to an alternative embodiment of the present invention, aptamer 3 is a DNA sequence comprising SEQ ID NO: 2. According to another embodiment of the present invention, the aptamer comprises a sequence with at least 30% identity to SEQ ID NO: 2, preferably at least 50%, more preferably 70%, even more preferably at least 90%.

According to an alternative embodiment of the present invention, aptamer 3 is a DNA sequence comprising SEQ ID NO: 3. According to another embodiment of the present invention, the aptamer comprises a sequence with at least 30% identity to SEQ ID NO: 3, preferably at least 50%, more preferably 70%, even more preferably at least 90%.

According to an alternative embodiment of the present invention, aptamer 3 is a DNA sequence comprising SEQ ID NO: 4. According to another embodiment of the present invention, the aptamer comprises a sequence with at least 30% identity to SEQ ID NO: 4, preferably at least 50%, more preferably 70%, even more preferably at least 90%.

Still according to the same configuration illustrated in Figure 1A, the phospholipids that make up the liposomal bilayer have their polar portion facing outwards from the bilayer (external portion 6 of the liposome and lumen 8 of the liposome) and the apolar portion facing the interior of the bilayer 7. According to a preferred configuration of the present invention, the bilayer 7 structure comprises in its apolar lumen at least one active compound.

According to a preferred embodiment of the present invention, the active compound comprised in the lumen of the bilayer 7 of the liposome 10 is a retinoic acid, more preferably ATRA, 9-cis RA and/or 13-cis RA. In this embodiment, the retinoic acid is present in the liposomal complex in a concentration between 0.001% and 0.4% by mass.

According to an alternative configuration, the active compound other than ATRA, 9-cis RA or 13-cis RA, can be chosen from the set of active compounds that have the capacity to induce the transformation of white adipocytes into brown adipocytes, including those listed in Table 1 of this descriptive report.

Advantages of the invention and preferred configuration

The present invention is the result of intense intellectual effort, using several techniques from different technological fields, in particular, liposomal technology, metabolism modulation and cell differentiation technology, targeted delivery of active compounds technology, aptamer technology, molecular conjugation technology, among others. The effort to connect and give functional cohesion to the numerous components and techniques that make the present invention possible is not a trivial task. In fact, technicians who master one of the technological aspects of the present invention would hardly be encouraged to seek contributions from the numerous technological fields from which the present invention benefits. Therefore, the present invention could not be considered a mere derivation of the state of the art.

In addition to revealing unprecedented liposomal complexes, the differential technical characteristics of the present invention are closely related to its superior technical effect related to the transformation of white adipocytes into brown ones, whether for therapeutic or cosmetic applications.

Additionally, the present invention provides a solution that can be incorporated into pharmaceutical and cosmetic compositions, resulting in compositions that are not only effective, but also with mitigated or even absent side effects.

EXAMPLES

1. Preparation of all-trans retinoic acid (ATRA)

The ATRA reagent (all trans retinoic acid, Sigma, St. Louis, MO) 20 mg will be dissolved directly in chloroform/methanol (1:1, v/v) in combination with the components for liposome formation. For the WAT to BAT transformation assays, 1.5 mg will be dissolved in 500 mL of dimethyl sulfoxide (DMSO) for preparation of stock at 10 μM and serial dilutions.

2. Preparation of aptamers

In parallel, the aptamer consisting of a sequence modified with carboxylic acid, amino group or thiol. The aptamers will be obtained, lyophilized and eluted to 100 μM according to the absorbances after synthesis and purification reported by the manufacturer. After elution, the aptamers will be quantified with the aid of a spectrophotometer and evaluated by electrophoresis in 4% agarose gel.

3. Preparation of liposomes

The liposomes will be composed of soybean phosphatidylcholine, cholesterol and cationic lipid in PBS buffer pH 7.4, EDC (1-ethyl-3-(3-dimethylaminopropyl carbodiimide)) and N-hydroxysuccinimide. Together with retinoic acid and aptamers, the components will be rotary evaporated, and the particles will be formed by extrusion. The encapsulation efficiency of the liposomal system will be determined by centrifugation of the sample in filters with pores of defined size, and molecular mass and determination of the retinoic acid concentration will be by UV-Vis spectrophotometry. The average diameter of the chosen sample will be determined by dynamic light scattering.

3.1 Alternative liposome preparation

The liposomal vesicle formulation was composed of soybean phosphatidylcholine (SPC), cholesterol (COL), 1,2-dioleoyl-3-trimethylammonium-propane (DOTAP) (SPC:COL:DOTAP) in the proportions of 0.45:0.3:0.25, and SPC:COL:DOTAP plus cholesterol hemisuccinate (CHEMS), in the proportion of 0.45:0.2:0.1:0.25 (SPC:COL:CHEMS:DOTAP), both containing ATRA at a concentration of 0.1 mM. After preparation, the balloons containing the formulations were subjected to rotary evaporation to form a lipid film. Then, the film was hydrated with distilled water and kept in an ultrasonic bath for complete suspension of the lipid film. The liposome was sonicated (Q500, QSONICA) at 30% power for 40 minutes, 1 minute ON and 1 minute OFF. At the end, the sample particles had their size measured by dynamic light scattering (DLS, Litesizer 500, ANTON PAAR) to determine the distribution and average size of the particles in dispersion.

4. Cultivation of cell lines

The murine preadipocyte cell line 3T3-L1 will be purchased cryopreserved from Sigma. The cells will be thawed using Dulbecco's modified Eagle's medium (DMEM) supplemented with 10% Fetal Calf Serum (FCS) and maintained in T-25 flasks with pH adjusted to 7.4 in an incubator at 37°C and 5% CO2. After three hours, upon cell adhesion, the medium will be removed, replaced with fresh medium and the flasks will remain in the incubator, with the medium being changed every two to three days. After reaching confluency of cell growth in the culture flask, the cells will be washed with phosphate saline solution and enzymatically deadhered with 0.05% trypsin and 0.02% EDTA in phosphate buffered saline solution. Immediately, the cells will be subcultured with fresh medium in T-75 flasks. Upon reaching 70-80% confluence, the cells will be successively subcultured with 2 to 3 x 103 cells/cm2 for stock production.

5. Differentiation of white adipocyte cells

Two days after 3T3-L1 cells reach near maximal confluence, referred to as day zero, cells will be induced to differentiate in DMEM supplemented with 10% Fetal Bovine Serum (FBS), 1 μM dexamethasone, 0.5 mM methyl isobutyl xanthine, and 1 μg/L insulin for 48 hours, followed by 48 hours in DMEM supplemented with 10% FBS and 1 μg/ml insulin. Cells will then be fed every 48 h with fresh medium without insulin. After differentiation, expected between seven and eight days, of preadipocytes into mature white adipocytes, cells will be evaluated using Oil Red O stain for intracellular lipid accumulation by solubility in 100% isopropanol and microplate reading at 510 nm and immunofluorescence micrographic imaging (Mercader et al. 2007).

6. Intracellular lipid analysis

Preadipocytes and adipocytes will be washed twice with PBS 1X (pH 7.4) and fixed with 10% paraformaldehyde for 30 minutes at room temperature. Fixed cells will be washed twice with distilled water and incubated with 60% isopropanol for 5 minutes. After removing the isopropanol, Oil Red O (ORO, Sigma-Aldrich) solution will be added and the plate incubated for 30 minutes at room temperature. After staining, the cells will be washed five times with distilled water and counterstained with Harris hematoxylin for 1 minute. After removing the hematoxylin, the cells will be washed 5 times and visualized under an optical microscope. To quantify fat droplets, stained cells will be solubilized with 100% isopropanol and optical density will be measured at 510 nm (Chung et al. 2021; Kim et al. 2014; Xue et al. 2022).

7. Transformation into brown adipocyte cells

After differentiation into white adipose cells, the cell population will be treated with concentrations prepared by serial dilution with a final concentration of 0.1, 0.2, 1, 2, 10 $\mu$M of ATRA or only vehicle composed of DMSO for five days. In the same period, treatment of the cell population with equivalent concentrations of rosiglitazone (positive control) will also be initiated. The medium containing the retinoic acid solution or DMSO will be exchanged for fresh medium every 24-48 hours. After 6 days, the membrane content of lipid droplets and mitochondrial mass will be evaluated (Mercader J. et al, 2007; Kiefer FW et al., 2012).

8. Lactate dehydrogenase cytotoxicity assay

Cell populations treated with ATRA, rosiglitazone or DMSO will be plated in flat-bottomed microtiter plates with 50 $\mu$l of RPMI and 3% FCS. The plates will then be incubated at 37°C and 5% CO2. After the incubation period, the microtiter plates will be removed from the incubator and kept for 5-10 minutes at room temperature. Then, 15 $\mu$l of the lysis solution will be added, and the plates will be centrifuged at 250g for 4 minutes. 50 $\mu$l of the supernatant will be transferred to a 96-well flat-bottomed enzyme assay plate. LDH activity will be measured based on the oxidation of NADH+ when pyruvate is converted to lactate. The enzymatic reaction will be initiated by adding 50 $\mu$l of freshly prepared NADH solution per well. For the standard curve, 50 $\mu$l of the substrate solution will be added to 50 $\mu$l and different known concentrations of LDH. Incubate the plate for 15-30 minutes at 37°C, protected from light. After the incubation period, 100 $\mu$L of Stop solution will be added. The optical density will be measured at 490 nm within 1 hour after the addition of the Stop solution. The background optical density will be measured at 690 nm and this value will be subtracted from the values of the 490 nm wavelength. The percentage of dead cells will be determined using the following equation (Kumar et al. 2018; Decker and Lohmann-Matthews 1988).

$$\% \; of \; Cytotoxicity = \frac{\text{LDH released by treated cells(490 nm)}}{\text{LDH released by control cells (490 nm)}}$$

9. Analysis of mitochondrial mass and membrane lipid quotients by confocal microscopy

The differentiated cell population will be treated with retinoic acid, rosiglitazone (positive control) or DMSO (negative control) solution in an 8-well cell culture chamber on a glass microscope slide filled with culture medium. After the treatment period, the cells will be incubated with 100 nM MitoTracker Green FM in Krebs-Ringer solution (125 mM sodium chloride, 5 mM potassium chloride, 1.3 mM calcium chloride, 1.2 mM magnesium sulfate, 25 mM HEPES adjusted to pH 7.4 and 2% BSA) for 20-40 minutes at 37°C. After the incubation period, the cells will be fixed with 4% paraformaldehyde, permeabilized with a 0.1% Triton X solution in PBS and incubated in 2% BSA solution in PBS for 1 hour at room temperature. Treated cells will be incubated with a primary antibody solution, anti-perilipin, and incubated overnight at 4°C. After the incubation period, cells will be washed with PBS and incubated with the secondary antibody conjugated with Alexa Fluor 647 for 1 hour at room temperature. Nuclei will be stained with 1 $\mu$g/ml DAPI for 5 minutes. Stacking images in the z-direction will be performed by confocal fluorescence microscopy measuring fluorescence intensity with excitation of 594/633 for perilipin and 492/517 nm for MitoTracker (Wilson-Fritch et al. 2003; Chazotte B. 2011; Harris LA, Skinner JR, Wolins NE, 2013).

10. Oxygen consumption

The oxygen consumption of the differentiated cell population treated with retinoic acid, rosiglitazone or DMSO solution will be determined by high-resolution respirometry (OROBOROS Instruments Corp., Innsbruck, Austria) at the Laboratory of Trypanosome Biochemistry, Institute of Biomedical Sciences, USP. Cells will be incubated in 2 mL of BIOPS buffer, at 4°C, with 50 $\mu$g/mL saponin for 30 minutes. After permeabilization, the cells will be washed twice for 5 minutes each in respiration medium consisting of 320 mM sucrose, 1 mM EGTA, 4 mM MgCl2, 5 mM KH2PO4, 10 mM TrisHCl and 1 mg/mL BSA/FF. After washing, the cells will be inserted into Oroboros oxygraph chambers with 2 mL of mitochondrial respiration medium at 37°C. To monitor the oxygen flux, the substrates will be added in the respective order: 5 mM pyruvate, 2 mM ADP, 10 mM succinate, 2 $\mu$M oligomycin and 1 $\mu$M antimycin A. The data will be extracted and analyzed by DatLab 4 software (Cordero-Reyes et al. 2014; Hughey et al. 2011; Wang et al. 2017).

11. Skin permeation

Functionalized retinoic acid will be prepared and evaluated by studying solubility, stability and dosage in the epidermal and dermal layers through a bioanalytical method based on HPLC coupled to a diode array detector (HPLC-DAD). A validation of the method will be performed through the selectivity and linearity parameters obtained.

12. Treatment of adipocytes with aptamers and verification of binding

This analysis aims to evaluate the binding capacity of aptamers in transformed white adipocytes.

Materials and methods

Cells were morphologically analyzed and micrographically recorded using an Evos XL Core inverted microscope (AMEX1000, Invitrogen, USA) and AxioVert.A1 (Zeiss, Germany).

Treatment of white adipocytes with aptamers

White adipocyte cells were treated with aptamers SEQ ID NO: 1, SEQ ID NO: 3, and their respective controls SEQ ID NO: 4 and SEQ ID NO: 5, containing Cy5 fluorophore labeling, according to the protocol previously established by Chen and collaborators in 2015. After three washes with wash buffer (5 mmol/L MgCl2 [C2014.01.AG, Lab Synth, Lot 257030] and 4.5 g/L glucose [G1008.01.AF, Lab Synth, Lot 258311] in DPBS 1x [141 90-144, Gibco, Lot 2430505]), the cells were incubated for 40 minutes with binding buffer (1 mg/L bovine serum albumin [BSA - Sigma-Aldrich , CAS 9048-46-8, Lot SLBP1160V] diluted in wash buffer) containing 125 nmol or 250 nmol of aptamer, at 37°C in the dark. Then, the cells were washed with binding buffer and incubated for 30 min with 4% paraformaldehyde (158127, STBK5668, Sigma-Aldrich) at room temperature. Subsequently, paraformaldehyde was removed, cells were washed and transferred to microscopy slides containing fluroshield containing DAPI and analyzed by fluorescence microscopy.

Fluorescence microscopy

Cells were analyzed and recorded micrographically using an AxioVert.A1 fluorescence microscope (Zeiss, Germany).

Results

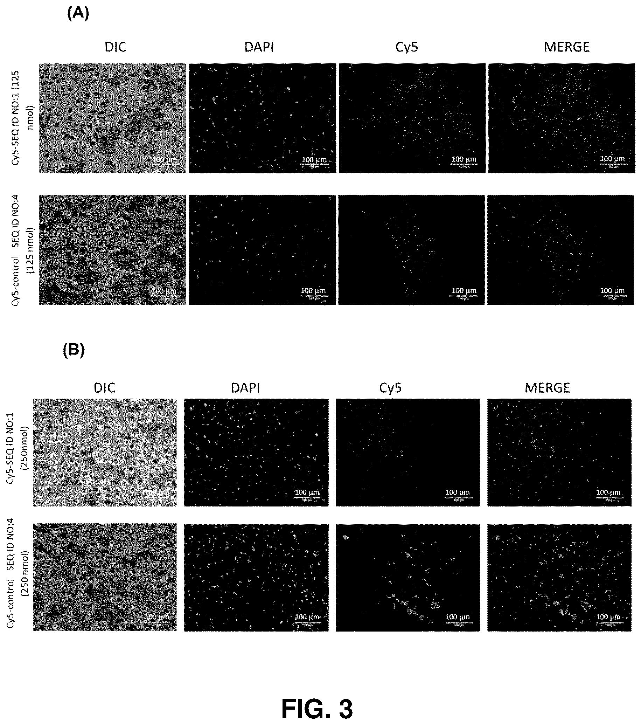

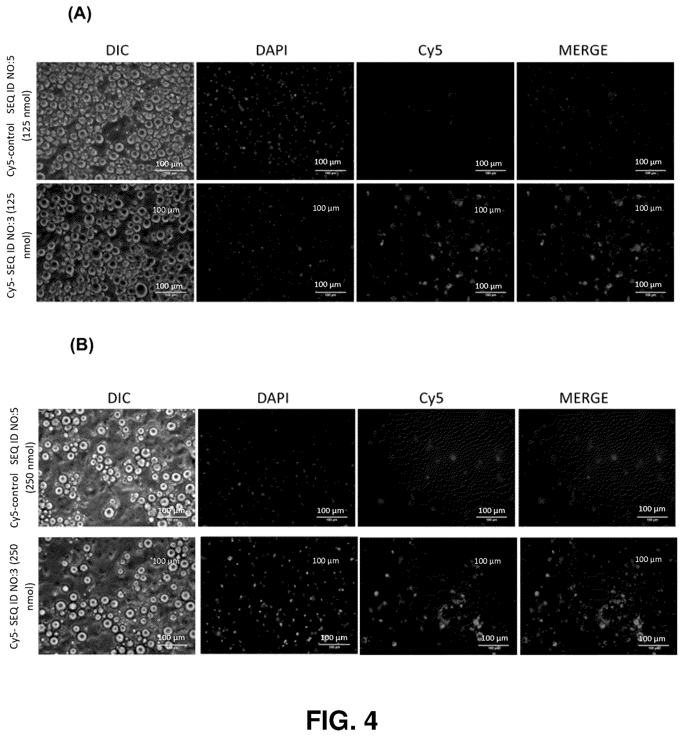

Aptamer binding analysis was performed on 3T3-L1 preadipocytes (ATCC) induced to adipogenic differentiation for 21 days. After the differentiation period, white adipocytes were treated with different concentrations of aptamers and analyzed by fluorescence microscopy (Figures 3 and 4), highlighting the cell nuclei labeled with DAPI, in blue, and the red fluorescence, originating from the interaction of the Cy5 marker with the cells. Cells treated with aptamers SEQ ID NO: 1 or SEQ ID NO: 3 showed higher red fluorescence intensity when compared to their respective controls (SEQ ID NO: 4 and 5). Furthermore, the increase in fluorescence was proportional to the aptamer concentration used: (A) 125 nM, (B) 250 nM. In parallel, it was also possible to observe an accumulation of red fluorescence in the plasma membrane of cells with rounded morphology, indicating that the aptamers under study have greater affinity with the white adipocyte cell model.

**Claims**

1. Liposomal complex wherein it comprises:

   a liposome (10), wherein said liposome (10) comprises a bilayer structure formed by amphiphilic molecules (2), wherein said amphiphilic molecules (2) comprise at least one phospholipid, at least one cholesterol and at least one cationic and/or ionizable lipid;
   at least one active compound (4);
   at least one targeting agent (3) coupled to the external portion of the liposome (6), wherein said at least one targeting agent is an aptamer (3).

2. Liposomal complex, according to claim 1, wherein the aptamer (3) has a sequence with at least 50% identity in relation to the sequence SEQ ID NO: 1.

3. Liposomal complex, according to claim 1, wherein the aptamer (3) has a sequence with at least 50% identity in relation to the sequence SEQ ID NO: 2.

4. Liposomal complex, according to claim 1, wherein the aptamer (3) has a sequence with at least 50% identity in relation to the sequence SEQ ID NO: 3.

5. Liposomal complex, according to claim 1, wherein the aptamer (3) has a sequence with at least 50% identity in relation to the sequence SEQ ID NO: 4.

6. Liposomal complex, according to any one of the preceding claims, wherein it additionally comprises a permeability agent.

7. Liposomal complex, according to claim 6, wherein the at least one permeability agent is an aptamer selected from a group of aptamers comprising SEQ ID NO: 6, SEQ ID NO: 7, SEQ ID NO: 8, SEQ ID NO: 9.

8. Liposomal complex, according to any one of the preceding claims, wherein the aptamer (3) comprises a terminal modification consisting of the addition of a NH2 and/or thiol (SH) group.

9. Liposomal complex, according to any one of the preceding claims, wherein the liposome (10) comprises the following amphiphilic molecules (2):
phosphatidylcholine or dioleoylphosphatidylethanolamine (DOPE), cholesterol and cationic or ionizable lipid in the proportion of 15-35:30-50:25-45 (w/w).

10. Liposomal complex, according to any one of the preceding claims, wherein it comprises soy phosphatidylcholine or dioleoylphosphatidylethanolamine (DOPE), cholesterol and dioleoyl-trimethylammonium propane or analogues of SM102, with or without the addition of 2% DMG-PEG 2000.

11. Liposomal complex, according to any one of the preceding claims, wherein the amphiphilic molecules comprised in the liposome (10) bilayer present a modification consisting of the addition of a COOH group to its phospholipid portion, and/or DMG-PEG 2000.

12. Liposomal complex, according to any one of the preceding claims, wherein the at least one active compound is selected from a group of retinoids.

13. Liposomal complex, according to claim 8, wherein the at least one active compound (4) is an all-trans retinoic acid (ATRA), 9-cis retinoic acid (9-cis RA) or 13-cis retinoic acid (13-cis RA).

14. Liposomal complex, according to claim 8 or 9, wherein the active compound (4) is present in the liposome (10) in a concentration between 0.001% and 0.4% by mass.

15. Liposomal complex, according to any one of the preceding claims, wherein the active compound (4) is located in the lumen of the bilayer (7) of the liposome (10).

16. Pharmaceutical composition wherein it comprises a liposomal complex as defined by any of the previous claims and at least one pharmaceutically acceptable excipient.

17. Cosmetic composition wherein it comprises a liposomal complex as defined by any one of claims 1 to 15, and at least one cosmetically acceptable excipient.

18. Use of the liposomal complex, as defined by any one of claims 1 to 15, wherein it is for the preparation of a medicine for the treatment of disorders and diseases related to the accumulation of white adipose tissue, including obesity, type 2 diabetes (T2DM), heart disease, metabolic syndrome, hyperglycemia, hypertension, steatosis and some types of cancer, such as endometrial, breast, esophageal and pancreatic cancer.

19. Use of the liposomal complex, as defined by any one of claims 1 to 15, wherein it is for the preparation of a cosmetic composition for reducing measurements.

FIG. 1

FIG. 2

FIG. 3

**FIG. 4**

## INTERNATIONAL SEARCH REPORT

| International application No. |
| --- |
| **PCT/BR2023/050490** |

| **A.** | **CLASSIFICATION OF SUBJECT MATTER** |
| --- | --- |

A61K 9/127 (2006.01)i; A61K 31/07 (2006.01)i; A61K 47/26 (2006.01)i; C12N 15/00 (2006.01)i; A61K 47/30 (2006.01)i; A61P 3/04 (2006.01)i; A61P 35/00 (2006.01)i

CPC:A61K9/1271,A61K9/1272,A61K31/07,A61K47/26,C12N15/115,A61K47/30

According to International Patent Classification (IPC) or to both national classification and IPC

| **B.** | **FIELDS SEARCHED** |
| --- | --- |

Minimum documentation searched (classification system followed by classification symbols)

A61K 9/127; A61K 31/07; A61K 47/26; C12N 15/00; A61K 47/30; A61P 3/04; A61P 35/00
CPC:A61K9/1271,A61K9/1272,A61K31/07,A61K47/26,C12N15/115,A61K47/30

Documentation searched other than minimum documentation to the extent that such documents are included in the fields searched

Banco de dados INPI-BR; Periódicos Capes; Google.

Electronic data base consulted during the international search (name of data base and, where practicable, search terms used)

REGISTRY e CAPLUS (STN); Derwent SequenceBase.

| **C.** | **DOCUMENTS CONSIDERED TO BE RELEVANT** |
| --- | --- |

| Category* | Citation of document, with indication, where appropriate, of the relevant passages | Relevant to claim No. |
| --- | --- | --- |
| X<br><br>Y | Kim, Minhee & Lee, Jong & Kim, Wooyeon & Lee, Jong & Jun, Bong-Hyun & Kim, Keun-Sik & Kim, Dong-Eun. (Available online 29 June 2022). Aptamer-conjugated nano-liposome for immunogenic chemotherapy with reversal of immunosuppression. Journal of controlled release : official journal of the Controlled Release Society. 348. 10.1016/j.jconrel.2022.06.039. See abstract and pages 894-895 | 1-6, 8, 10, 11, 14, 15-18<br><br>7, 9, 12, 13, 19 |
| Y | Cuomo, Francesca & Ceglie, Sara & Miguel, Maria & Lindman, Bjorn & Lopez, Francesco. (2021). Oral delivery of all-trans retinoic acid mediated by liposome carriers. Colloids and surfaces B: Biointerfaces. 201. 111655. 10.1016/j.colsurfb.2021.111655. See abstract; pages 2 and 4; figure 3 | 1-19 |
| Y | WO 2013135800 A1 (BIONEER AS  [DK]) 19 September 2013 (2013-09-19)<br>See abstract; claims 1, 8-14, 16, 29; figure 1 | 1-19 |

| ☑ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **14 March 2024** | **26 March 2024** |

| Name and mailing address of the ISA/BR | Authorized officer |
| --- | --- |
| **National Institute of Industrial Property (Brazil)**<br>**Rua Mayrink Veiga, 9, 6º andar, CEP 20.090-910 Rio de Janeiro – RJ**<br>**Brazil** | **Rodinelli OLIVEIRA** |
| Telephone No. **(55 21) 3037-3742, 3037-3984** | Telephone No. **+55 21 3037 4528 - 3037 3319** |

Form PCT/ISA/210 (second sheet) (July 2022)

## INTERNATIONAL SEARCH REPORT

| International application No. |
| --- |
| **PCT/BR2023/050490** |

**C. DOCUMENTS CONSIDERED TO BE RELEVANT**

| Category* | Citation of document, with indication, where appropriate, of the relevant passages | Relevant to claim No. |
| --- | --- | --- |
| Y | Liu J, Liu H, Sefah K, Liu B, Pu Y, Van Simaeys D, Tan W. Selection of aptamers specific for adipose tissue. PLoS One. 2012;7(5):e37789. doi: 10.1371/journal.pone.0037789. Epub 2012 May 25. PMID: 22662223; PMCID: PMC3360593. See the whole document, especially the abstract and tables 1 and 2 | 1-19 |
| Y | CN 112587670 A (UNIV CHINA AGRICULTURAL) 02 April 2021 (2021-04-02)<br>    See abstract; paragraph 0006; claims | 1-19 |
| Y | Kim EY, Kim JW, Kim WK, Han BS, Park SG, Chung BH, Lee SC, Bae KH. Selection of aptamers for mature white adipocytes by cell SELEX using flow cytometry. PLoS One. 2014 May 20;9(5):e97747. doi: 10.1371/journal.pone.0097747. PMID: 24844710; PMCID: PMC4028271. See the whole document, especially the abstract, table 1 and page 6 | 1-19 |
| Y | Duan T, Xu Z, Sun F, Wang Y, Zhang J, Luo C, Wang M. HPA aptamer functionalized paclitaxel-loaded PLGA nanoparticles for enhanced anticancer therapy through targeted effects and microenvironment modulation. Biomed Pharmacother. 2019 Sep;117:109121. doi: 10.1016/j.biopha.2019.109121. Epub 2019 Jun 25. PMID: 31252265. See abstract and page 2 | 1-19 |
| Y | KR 20170122653 A (UNIV SEJONG IND ACAD COOP FOUD  [KR]) 06 November 2017 (2017-11-06)<br>    See figures 1, 10 and 11; paragraphs 0025, 0058, 0114-0125 | 1-19 |
| Y | WO 2020010059 A1 (APTAMIR THERAPEUTICS INC  [US]) 09 January 2020 (2020-01-09)<br>    See abstract and paragraph 0010 | 1-19 |
| Y | Daniel C. Berry & Noa Noy (2009) All-trans-Retinoic Acid Represses Obesity and Insulin Resistance by Activating both Peroxisome Proliferation-Activated Receptor β/δ and Retinoic Acid Receptor, Molecular and Cellular Biology, 29:12, 3286-3296, DOI: 10.1128/ MCB.01742-08. See the whole document, especially the abstract and page 3295 | 1-19 |
| X<br>Y | WO 2018170398 A1 (CHILDRENS MEDICAL CENTER  [US]) 20 September 2018 (2018-09-20)<br>    See abstract; pages 2-4, 11-15, 29, 30, 33; claims 14, 24 | 1-6, 8, 10, 11, 14, 15-18<br>7, 9, 12, 13, 19 |
| X<br>Y | KR 101085203 B1 (SNU R AMP DB FOUNDATION) 21 November 2011 (2011-11-21)<br>    See paragraphs 0010-0018, 0027; example 6; comparative example 5; comparative example 11; examples 7, 8, 10-11, 15, 16, 19, 20; comparative examples 39 and 40 | 1-6, 8, 10, 11, 14, 15-18<br>7, 9, 12, 13, 19 |
| X<br>Y | Manoochehri H, Jalali A, Tanzadehpanah H, Taherkhani A, Najafi R. Aptamer-conjugated nanoliposomes containing COL1A1 siRNA sensitize CRC cells to conventional chemotherapeutic drugs. Colloids Surf B Biointerfaces. Available online 21 july 2022 Oct;218:112714. doi: 10.1016/j.colsurfb.2022.112714. Epub 2022 Jul 21. PMID: 35905589. See abstract and pages 2 and 3 | 1-6, 8, 10, 11, 14, 15-18<br>7, 9, 12, 13, 19 |
| X<br>Y | Moitra P, Misra SK, Kumar K, Kondaiah P, Tran P, Duan W, Bhattacharya S. Cancer Stem Cell-Targeted Gene Delivery Mediated by Aptamer-Decorated pH-Sensitive Nanoliposomes. ACS Biomater Sci Eng. 2021 Jun 14;7(6):2508-2519. doi: 10.1021/acsbiomaterials.1c00110. Epub 2021 Apr 19. PMID: 33871960. See abstract; figure 1; pages 2509 and 2510 | 1-6, 8, 10, 11, 14, 15-18<br>7, 9, 12, 13, 19 |

Form PCT/ISA/210 (second sheet) (July 2022)

## INTERNATIONAL SEARCH REPORT

| International application No. |
| --- |
| **PCT/BR2023/050490** |

### C. DOCUMENTS CONSIDERED TO BE RELEVANT

| Category* | Citation of document, with indication, where appropriate, of the relevant passages | Relevant to claim No. |
| --- | --- | --- |
| Y | Kitagawa, Shuji & Kasamaki, Masatoshi. (2006). Enhanced Delivery of Retinoic Acid to Skin by Cationic Liposomes. Chemical & pharmaceutical bulletin. 54. 242-4. 10.1248/cpb.54.242. See abstract and page 242 | 1-19 |
| Y | KR 20150058108 A (KOREA RES INST OF BIOSCIENCE [KR]) 28 May 2015 (2015-05-28) See abstract and pages 9 and 12 | 1-19 |
| Y | KR 101550721 B1 (KOREA RES INST OF BIOSCIENCE) 07 September 2015 (2015-09-07) See abstract; pages 9-12; claims | 1-19 |
| Y | Xue T, Xu H, Du Y, Ding J, Su Y, Lin Z. Browning of white adipocytes by gold nanocluster mediated electromagnetic induction heating hyperthermia. Nanoscale. Pub Date: 2021-12-13;14(4):1187-1194. doi: 10.1039/d1nr07263c. PMID: 35005765. Electronic Supplementary Information (ESI). https://www.rsc.org/suppdata/d1/nr/d1nr07263c/d1nr07263c1.pdf See abstract and tables S1 and S2 | 1-19 |
| Y | Shahdadi, Hossein & Jebali, Ali & Iman, Maryam. (2019). Dual function of interleukin-23 Aptamer to suppress brain inflammation via attachment to macrophage stimulating 1 kinase and interleukin-23. Colloids and Surfaces B: Biointerfaces. 185. 110619. 10.1016/j.colsurfb.2019.110619. See abstract and pages 2 and 5 | 1-19 |

Form PCT/ISA/210 (second sheet) (July 2022)

## INTERNATIONAL SEARCH REPORT

International application No.

**PCT/BR2023/050490**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

| INTERNATIONAL SEARCH REPORT<br>Information on patent family members | | | | International application No.<br>**PCT/BR2023/050490** | | | |
|---|---|---|---|---|---|---|---|
| Patent document<br>cited in search report | | | Publication date<br>(day/month/year) | Patent family member(s) | | | Publication date<br>(day/month/year) |
| WO | 2013135800 | A1 | 19 September 2013 | AU | 2013231261 | A1 | 16 October 2014 |
| | | | | CA | 2867060 | A1 | 19 September 2013 |
| | | | | CN | 104703588 | A | 10 June 2015 |
| | | | | EP | 2638896 | A1 | 18 September 2013 |
| | | | | EP | 2825158 | A1 | 21 January 2015 |
| | | | | JP | 2015509968 | A | 02 April 2015 |
| | | | | US | 2015079155 | A1 | 19 March 2015 |
| CN | 112587670 | A | 02 April 2021 | CN | 112587670 | B | 11 May 2021 |
| KR | 20170122653 | A | 06 November 2017 | KR | 101971451 | B1 | 14 August 2019 |
| WO | 2020010059 | A1 | 09 January 2020 | CA | 3105394 | A1 | 09 January 2020 |
| | | | | CN | 112805004 | A | 14 May 2021 |
| | | | | EP | 3817747 | A1 | 12 May 2021 |
| | | | | EP | 3817747 | A4 | 06 July 2022 |
| | | | | JP | 2021531252 | A | 18 November 2021 |
| | | | | KR | 20210044771 | A | 23 April 2021 |
| | | | | US | 2021145745 | A1 | 20 May 2021 |
| | | | | US | 11690804 | B2 | 04 July 2023 |
| WO | 2018170398 | A1 | 20 September 2018 | CA | 3056797 | A1 | 20 September 2018 |
| | | | | EP | 3595636 | A1 | 22 January 2020 |
| | | | | EP | 3595636 | A4 | 13 January 2021 |
| | | | | US | 2020085972 | A1 | 19 March 2020 |
| | | | | US | 11260132 | B2 | 01 March 2022 |
| KR | 101085203 | B1 | 21 November 2011 | CN | 112587670 | B | 11 May 2021 |
| KR | 20150058108 | A | 28 May 2015 | KR | 101545183 | B1 | 19 August 2015 |
| KR | 101550721 | B1 | 07 September 2015 | NONE | | | |

Form PCT/ISA/210 (patent family annex) (July 2022)